# EUROPEAN PATENT SPECIFICATION

(11) **EP 1 762 185 B1**
(45) Date of publication and mention of the grant of the patent: **10.12.2008**
(21) Application number: 06013489.7
(22) Date of filing: 29.06.2006
(51) Int. Cl.: A61B 17/04, A61B 17/06, A61B 17/12

(54) **Treating implement cartridge for living body tissue**
Patrone für eine Behandlungseinrichtung für lebendes Körpergewebe
Cartouche d'équipement de traitement de tissu organique vivant

(30) Priority: 13.09.2005 US 225588
(43) Date of publication of application: 14.03.2007
(73) Proprietor: OLYMPUS MEDICAL SYSTEMS CORP., Tokyo (JP)
(72) Inventor: Onishi, Norio, Tokyo (JP); Miyamoto, Satoshi, Tokyo (JP); Sakamoto, Yuji, Tokyo (JP); Onuki, Yoshio, Tokyo (JP)
(74) Representative: von Hellfeld, Axel

(56) References cited:
- US-A1- 2003 236 535
- US-A1- 2005 149 067
- US-A1- 2005 288 710

## Description

### BACKGROUND OF THE INVENTION

### Field of the Invention

The present invention relates to a treating implement cartridge which is used with an endoscope, or the like, and accommodates a treating implement which can be mounted on a treating apparatus performing the treatment of a living body tissue.

### Description of Related Art

As treating apparatuses which is inserted into the interior of the body with an endoscope, and performs treatment, there are some in which a treating implement can be mounted on a front end of the treating apparatus. As such a treating apparatus, there is a ligature and suture system for medical application which is used for performing ligature and suture on living body tissue. The ligature and suture system for medical application is provided with a tissue through needle which penetrates into living body tissue, and a ligation implement which can independently protrude or recede with respect to the tissue through needle. As the treating implement in the above case, treating implement is used in which an engagement member is fixed to one end of the thread for seaming, and another end of the thread for seaming is press-fitted into the stopper (see, for example, Patent Document 1: Japanese Unexamined Patent Application, First Publication No. 2004-601). When the treating implement is mounted on the ligature and suture system for medical application, the engagement member is accommodated in the hollow needle of the tissue through needle, and the ligation thread is pulled out through the slit which is formed in the hollow needle of the tissue through needle. Furthermore, the loop which is formed at another end of the thread for seaming pulled out from the stopper is engaged with the hook which projects from the sheath of the ligation implement. Then, the hook is pulled back, the loop is pulled into the sheath, and the stopper of the treating implement is connected to the front end surface of the sheath of the ligation implement.

When ligature and suture of living body tissue are carried out, the living body tissue is penetrated from the front to the back by the tissue through needle, the engagement member is released at the back of the living body tissue, and then, the tissue through needle is pulled back. Thereby, because the thread for seaming penetrates into the living body tissue, the thread for seaming is pulled by the ligation implement, and the living body tissue is bound tight by the engagement member and the stopper.

United States Patent Application Publication No. US 2003/236535 A1 describes an endoscopic treatment apparatus incorporating lumens integrally formed in a wall of the endoscope sheath or channel through which a ligating implement may be advanced and deployed.

### SUMMARY OF THE INVENTION

The primary object of the present invention is to make it so that the treating implement (i.e. ligating implement) can be easily mounted on the treating apparatus.

The present invention provides a ligating implement cartridge for use in ligating living body tissue as defined in claim 1. Preferred features of the invention are recited in the dependent claims.

Thus, the present invention provides a ligating implement cartridge for living body tissue having a ligating implement which has a plurality of engaging portions engaged with a plurality of engaged portions which are provided at a front end portion of a treatment apparatus inserted into an interior of a body in order to perform treatment of the living body tissue, and performs the ligation treatment of the living body tissue according to operation of the treating apparatus by an operator, an accommodation container which has a plurality of accommodation portions which accommodate the engaging portions of the ligating implement, and have opening portions into which the engaged portions are inserted, and a fixing portion which is attached to at least one accommodation portion, and holds the engaging portion.

Moreover, in the present invention, a first engaging portion from among the plurality of engaging portions is engaged with a first engaged portion from among the plurality of engaged portions, a second engaging portion from among the plurality of engaging portions is engaged with a second engaged portion from among the plurality of engaged portions, and a flexible member which connects the first engaging portion and the second engaging portion is provided, in which, when the first engaged portion which is attached to the first engaging portion is relatively moved with respect to the second engaged portion, the second engaging portion is pulled via the flexible member, and an engagement state between the second engaging portion and the second engaged portion may be maintained.

In the present invention, the fixing portion may fix the first engaging portion in a direction in which the first engaged portion is pushed and engaged, and in an inverse direction thereof, and the accommodation container may fix the second engaging portion in a direction in which the second engaged portion is pushed and engaged.

In the present invention, the flexible member may be a thread for seaming, the second engaged portion a hollow needle by which the living body tissue is penetrated, and the second engaging portion an engagement member which is arranged in the inside of the hollow needle, and, after the hollow needle penetrates into the living body tissue, is released from the inside of the hollow needle, and makes the flexible member engage with the living body tissue.

In the present invention, an engagement portion which is engaged with the treating apparatus inserted into the accommodation portion may be provided at the opening portion of the accommodation container.

### BRIEF DESCRIPTION OF THE DRAWINGS

FIG. 1 is a view which shows a constitution of a ligature and suture system for medical application according to an embodiment of the present invention.
FIG. 2 is a perspective view of a front end portion of the ligature and suture system for medical application.
FIG. 3 is a cross-sectional view of the front end portion of the ligature and suture system for medical application, as seen from an arrow A in FIG. 2.
FIG. 4 is a cross-sectional view seen from an arrow B in FIG. 3.
FIG. 5 is a view seen from an arrow C in FIG. 4.
FIG. 6 is a partial ruptured view which shows a constitution of an operation portion.
FIG. 7 is a view which shows a constitution of the treating implement.
FIG. 8 is a perspective view which shows the treating implement cartridge.
Fig. 9 is a perspective view which shows the accommodation container of the treating implement cartridge.
FIG. 10 is a cross-sectional view of a groove along line D-D in FIG. 8.
FIG. 11 is a cross-sectional view of an elastic member for fixing of the accommodation container.
FIG. 12 is a view as seen from an arrow E in FIG. 9.
FIG. 13 is a cross-sectional view of a through hole along line F-F in FIG. 8.
FIG. 14 is a view which shows an example of a storage method of the treating implement cartridge.
FIG. 15 is a view which shows a using method of the treating implement cartridge.
FIG. 16 is a view which explains an operation when equipping the treating implement cartridge.
FIG. 17 is a view which shows a state in which the ligation implement is inserted into the treating implement cartridge.
FIG. 18 is a view which shows a state in which the ligation implement and the treating implement are engaged with each other.
FIG. 19 is a view in which a holding member of the treating implement is in contact with the ligation implement.
FIG. 20 is a view which shows a state in which a tissue through needle is inserted into the treating implement cartridge.
FIG. 21 is a view which explains an operation when the treating implement is pulled out from the accommodation container.
FIG. 22 is a view which shows a process of pulling out the treating implement from the accommodation container.
FIG. 23 is a view which shows a process in which an engagement member is accommodated in a hollow needle.
FIG. 24 is a view which explains operation.
FIG. 25 is a view in which the hollow needle is penetrating into a treatment target.
FIG. 26 is a view in which the engagement member is pushed out from the hollow needle.
FIG. 27 is a view in which the hollow needle is pulled back.
FIG. 28 is a view in which the ligation implement is operated, and the treatment target is bound with the thread for seaming.

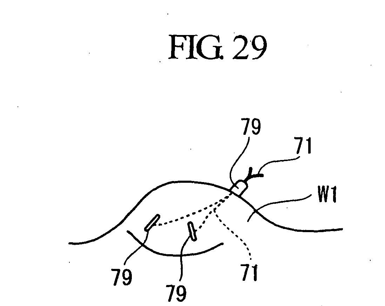

FIG. 29 is a view which shows the treatment target for which the ligature and the suture are performed.
FIG. 30 is a view which shows another embodiment of the treating implement cartridge.
FIG. 31 is a view which shows another embodiment of the treating implement cartridge.
FIG. 32 is a view which shows another embodiment of the treating implement cartridge.

### DETAILED DESCRIPTION OF THE INVENTION

An embodiment of the present invention is explained with reference to the figures.

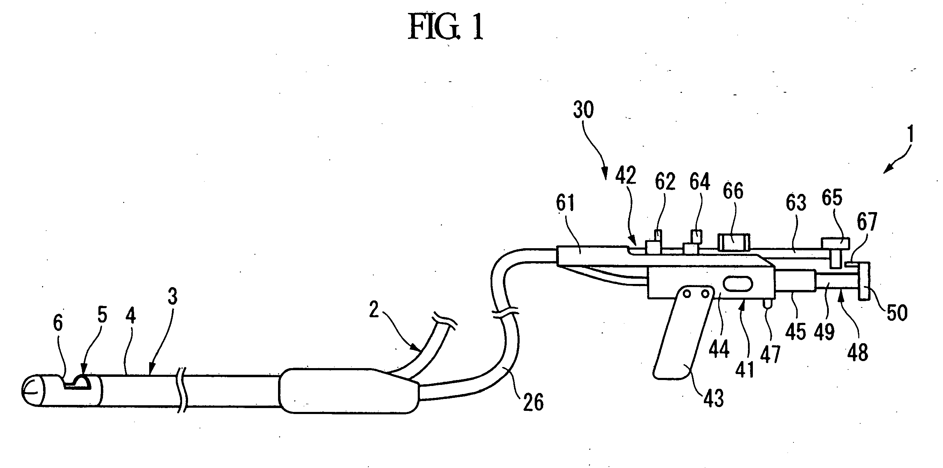

As shown in FIG. 1, a ligature and suture system for medical application 1 has a long over tube 3 into which an endoscope 2 can be inserted. In the over tube 3, a chamber 5 which is harder than a flexible tube body 4 is detachably attached to a front end of the tube body 4 (a distal end as seen from an operator). A side hole 6 is formed in a side portion of the chamber 5, and a living body tissue which is a treatment target can be pulled into the chamber 5 from the side hole 6.

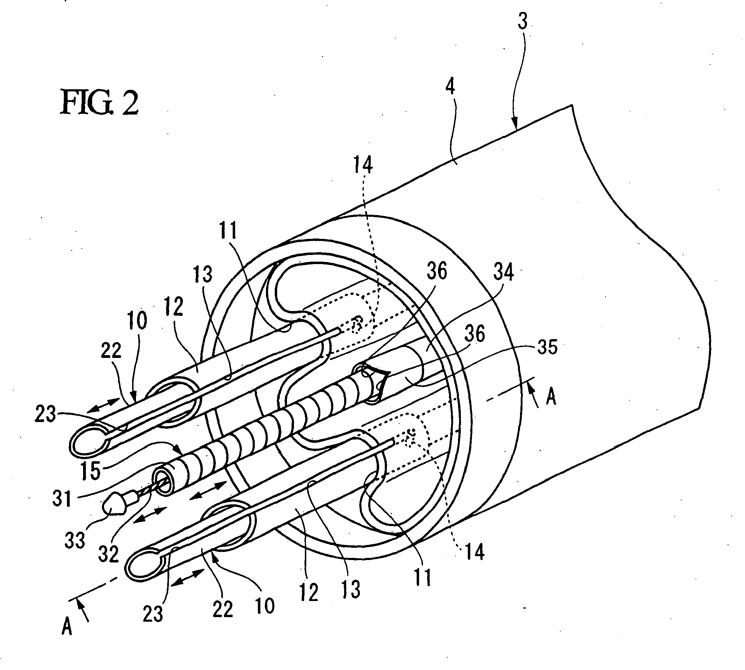

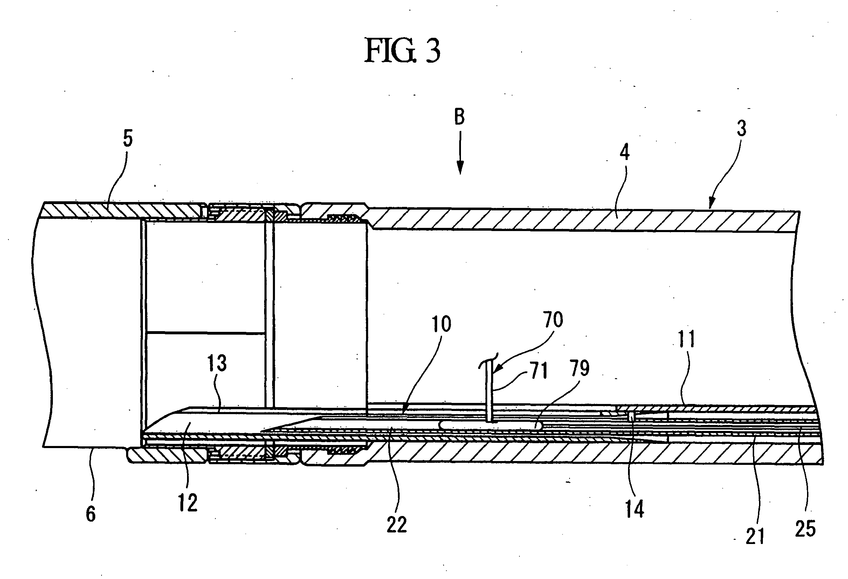

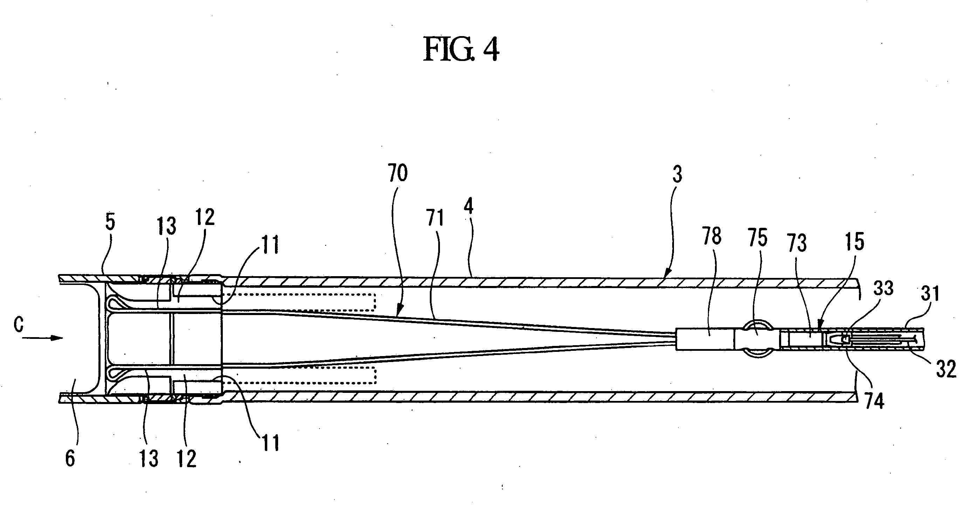

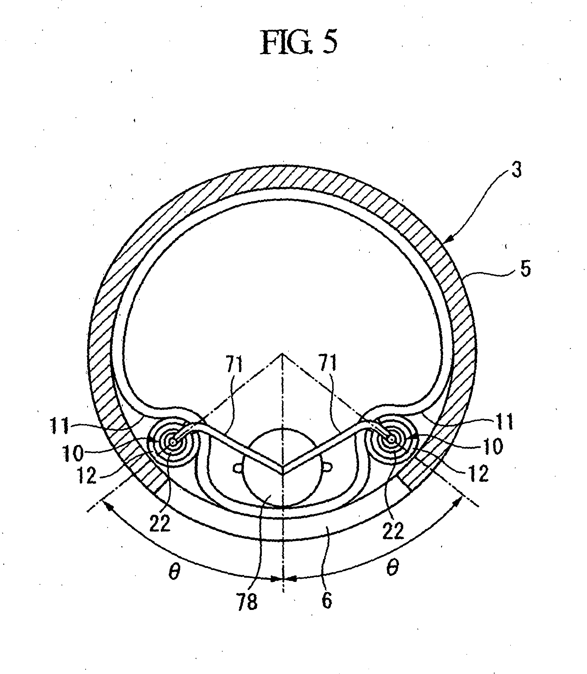

As shown in FIGS. 2 and 3, two needle lumens 11 through which two tissue through needles 10 run are formed on the inside of the tube body 4 of the over tube 3, and hard type needle guides 12 which project into the inside of the chamber 5 are fixed at the front end portion of each needle lumen 11. In the needle guides 12, a front end of a cylindrical member is cut at an angle, and a slit 13 is formed at a position which faces a central axis of the chamber 5. The slit 13 is extended to a predetermined length from the front end surface of the needle guide 12, and further, a needle positioning pin 14 projects toward the central axis of the needle guide 12 at the position corresponding to the base end portion of the needle guide 12 on the extending line of the silt 13. As shown in FIGS. 4 and 5, the ligation implement 15 is inserted into the space between the two tissue through needles 10 within the over tube 3. The angles between the ligation implement 15 and two tissue through needles 10, respectively, with respect to the standard of the central axis of the tube body 4 are a predetermined angle θ.

As shown in FIGS. 2 and 3, the tissue through needles 10 have flexible needle sheaths 21 inserted into the needle lumens 11, and the hollow needles 22 which are the second engaged portions are attached to the front ends of the needle sheaths 21. The hollow needles 22 are constituted by the hollow members which have inclined surfaces of which the front ends are sharp, and in which the slits 23 are formed from the front ends to the base ends thereof. The needle positioning pins 14 of the needle guides 12 are inserted into the slits 23, and thereby, the positions of the slits 23 of the hollow needles 22 and the positions of the slits 13 of the needle guides 12 are matched with each other. Furthermore, a pusher 25 is inserted into each needle sheath 21 and hollow needle 22, and the pusher 25 can be protruded or receded with respect to the needle sheath 21 and the hollow needle 22. The front end portions of the pushers 25 are prolonged to the front end sides beyond the needle positioning pins 14. That is, the formation and the projection amount of the needle positioning pins 14 are defined so that the protruding or receding of the pushers 25 is not prevented. The base end portions of the needle sheaths 21 and the pushers 25 are pulled out from the base end portion of the over tube 3, and are passed through the inside of the tube 26 which prolongs from the base end (near position end) of the over tube 3, as shown in FIG. 1, and are connected to a below-described operation portion 30.

As shown in FIG. 2, the ligation implement 15 has a flexible ligation sheath 31, and an operation wire 32 is inserted into the ligation sheath 31, while the operation wire 32 can be protruded or receded with respect to the ligation sheath 31. An arrowhead hook 33 which is a first engaged portion is attached to the front end portion of the operation wire 32. The arrowhead hook 33 has an outside diameter such that the arrowhead hook 33 can be accommodated in the ligation sheath 31. Furthermore, a sheath for cutting 34 is mounted on the ligation sheath 31 at the outer circumference of the ligation sheath 31, and the sheath for cutting 34 can be protruded or receded with respect to the ligation sheath 31. An annular cutting member 35 is attached to the front end portion of the sheath for cutting 34. A sharpened cutting portion 36 is formed on the front end of the cutting member 35. The base end portions of each sheath 31 and 34, and the operation wire 32 are pulled out from the base end of the over tube 3, are passed through the inside of the tube 26 shown in FIG. 1, and are connected to the operation portion 30.

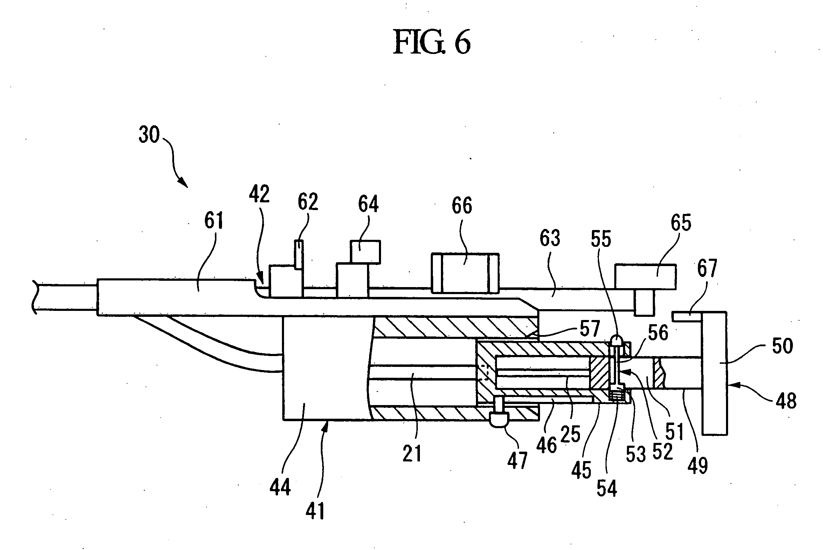

As shown in FIGS. 1 and 6, the operation portion 30 has a needle operation portion 41 operating the tissue through needles 10, and a ligation operation portion 42 operating the ligation implement 15 attached to the upper portion of the needle operation portion 41. The needle operation portion 41 has an accommodation portion 44 to which a grip 43 gripped by the operator is attached, a needle handle 45 is inserted into the inside of the accommodation portion 44 from the base end side thereof. The needle handle 45 has an elongated shape, and a groove 46 is formed in the outer circumference of the lower portion thereof. The front end portion of a pin 47 screwed in from the outer surface of the accommodation portion 44 is inserted into the groove 46, and the end portion of the groove 46 can be protruded or receded from the retracted position at which the end portion of the groove 46 is engaged with the pin 47 to the penetrating position, at which the front end surface of the needle handle 45 is abuts the inside surface of the accommodation portion 44.

Furthermore, a pusher handle 48 is inserted into the inside of the needle handle 45 from the base end side thereof. In the presssing handle 48, a large diameter of handle body 50 is provided at the base end of an elongated sliding portion 49 which can be inserted into the needle handle 45. A slit 51 running vertically from the front end side to the base end side is formed in the sliding portion 49, and a connection pin 52 supported at the base end side of the needle handle 45 is inserted into the slit 51. The base portion 53 of the connection pin 52 is urged toward the upper portion by an elastic member 54 such as a coil spring, or the like, and the front end portion 55 of the connection pin 52 projects from the upper surface of the needle handle 45 at a predetermined length in its natural state. The diameter of the intermediate portion 56 connecting the base portion 53 and the front end portion 55 of the connection pin 52 is smaller than the diameter of the base portion 53. Although the diameter of the intermediate portion 56 is not more than the width of the slit 51 of the pusher handle 48, the diameter of the base portion 53 of the connection pin 52 is larger than the width of the slit 51.

A notch which can receive the base portion 53 is formed in the front end portion of the slit 51, and, in the state in which the pusher handle 48 is pulled out to the maximum, the base portion 53 of the connection pin 52 which is urged toward the upper direction by the elastic member 54 is pulled into the slit 51, and the pusher handle 48 and the needle handle 45 are connected. On the other hand, when the front end portion 55 of the connection pin 52 is pushed back downward by the inclined surface 57 of the opening at the base end side of the accommodation portion 44, the base portion 53 of the connection pin 52 slips out from the slit 51, and the engagement between the connection pin 52 and the pusher handle 48 is released. By such a method, the pusher handle 48 can be independently protruded or receded with respect to the needle handle 45.

In addition, the two needle sheaths 21 of the tissue through needles 10 are inserted into the inside of the accommodation portion 44 from the front end portion thereof, and are fixed to the front end surface of the needle handle 45. Furthermore, the pushers 25 within the needle sheaths 21 are inserted into the inside of the needle handle 45 from the front end portion thereof, and are fixed to the front end surface of the pusher handle 48.

The ligation operation portion 42 is provided at a slide guide 61 which is integrally provided at the upper portion of the needle operation portion 41 so that the ligation operation portion 42 can slide with respect to the slide guide 61 from the base end side thereof. The ligation operation portion 42 has a cutting handle 62, and the base end portion of the sheath for cutting 34 is fixed to the cutting handle 62. Furthermore, the operation portion body 63 of the ligation implement 15 is inserted into the base end side against the cutting handle 62 so that the operation portion body 63 can be protruded or receded with respect to the slide guide 61 and the cutting handle 62. The ligation sheath 31 is fixed at the front end of the operation portion body 63. In the operation portion body 63, a knob portion 64 operated by the operator is provided at a position near the cutting handle 62. Furthermore, a finger control portion 65 on which the operator puts a finger and operates is provided at the base end portion of the operation portion body 63. Furthermore, the ligation handle 66 is attached to the operation portion body 63, and is protruded or receded along the length thereof. The base end portion of the operation wire 32 is fixed to the ligation handle 66. In addition, the engagement member 67 attached to the handle body 50 of the pusher handle 48 of the needle operation portion 41 can be engaged with the finger control portion 65 from the base end side.

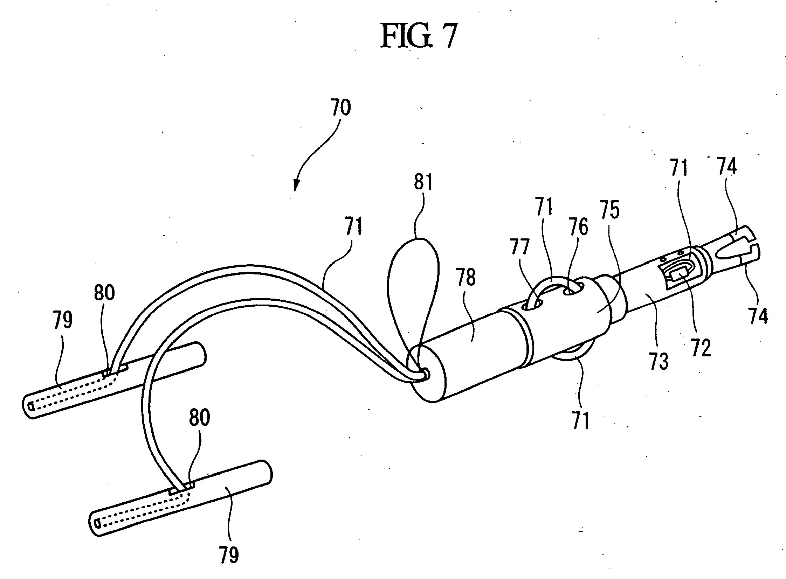

Next, with reference to FIG. 7, the treating implement 70 mounted on the front end portion of the ligature and suture system for medical application 1 is explained. The treating implement 70 has a thread 71 for seaming which is a flexible member. The thread 71 for seaming is folded over approximately at the center portion thereof, and an escape stop pipe 72 is inserted into the neighborhood of the folded over part, and the connection member 73 is fixed so that the escape stop pipe 72 is covered. The connection member 73 is a first engaging portion in which a pair of engaging pieces 74 which can be elastically deformed are extended from the base end portion thereof, and a hooked portion which can be engaged with the arrowhead hook 33 (see FIG. 2) is formed at the end portion of each engagement piece 74. The thread 71 for seaming pulled out from the connection member 73 is inserted into the inside of the holding member 75. In the holding member 75, the opening at the side of the base end is formed at the base end surface which is in contact with the connection member 73, a cylindrical member in which the front end opening is formed at the front end surface thereof is provided, and two side holes 76 and two side holes 77 are formed along the length thereof. The thread 71 for seaming is put into the inside of the holding member 75 from the opening at the base end side of the holding member 75, is pulled out from the side holes 76 to outside the holding member 75, is pulled back to the inside of the holding member 75 from the side holes 77, and then, is pulled out from the front end opening. The stopper 78 which is a fixing member made from silicone rubber or the like is press-fitted into the thread 71 for seaming which is pulled out in this state. Furthermore, each end portion of the thread 71 for seaming is inserted into an engagement member 79 from a slit 80 formed at approximately the center portion along the length of the elongated engagement member 79 as the second engaging portion, and is fixed by a method such as adhesion, fixing by pressure, or the like.

Furthermore, in FIG. 7, a reverse thread 81 is hooked on the thread 71 for seaming pulled out from the stopper 78 in the loop state. When, after the living body tissue is seamed by the below-described operation, the seaming state is released, the reverse thread 81 is used, and the reverse thread 81 is pulled by a gripping forceps, or the like, the stopper 78 is slidably moved to the base end side, and thereby the ligation can be released. In addition, such a reverse thread 81 is not a indispensable constitution in the treating implement 70 according to the present embodiment.

As shown in FIGS. 3 and 4, the treating implement 70 is mounted on the front end portions of the tissue through needles 10 and the front end portion of the ligation implement 15, and is used. The engagement member 79 is accommodated in the hollow needles 22 of the tissue through needles 10, and the thread for seaming 71 is pulled out from the slits 23 of the hollow needles 22. Moreover, the engagement piece 74 of the connection member 73 is engaged with the arrowhead hook 33 of the ligation implement 15, the connection member 73 is pulled into the inside of the ligation sheath 31, and the base end surface of the holding member 75 is brought in contact with the front end surface of the ligation sheath 31.

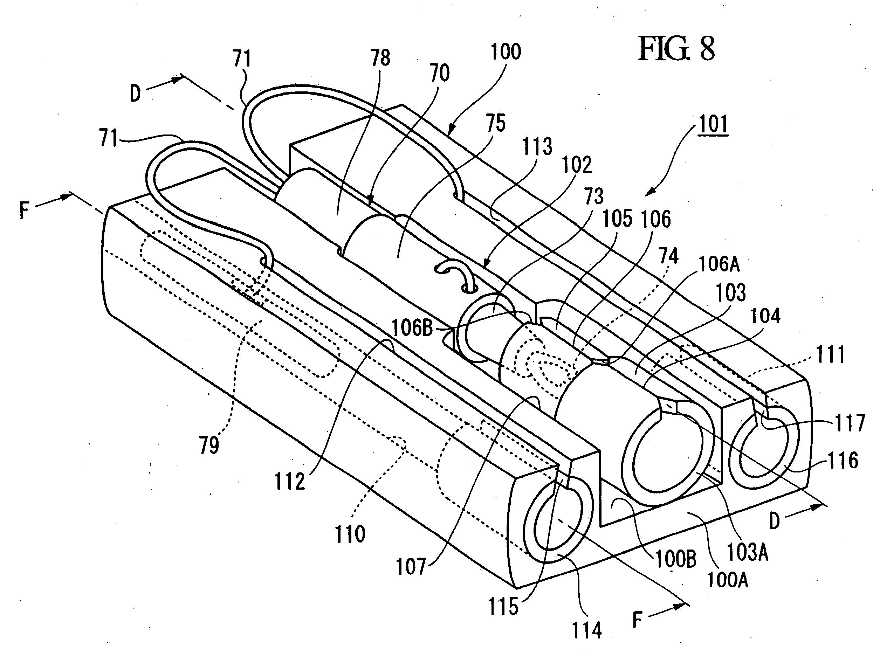

Here, as shown in FIG. 8, the treating implement 70 is kept as a treating implement cartridge 101 accommodated in an accommodation container 100. The treating implement cartridge 101 is constituted so that the treating implement 70 can be directly mounted on the front end portions of the tissue through needles 10 and the front end portion of the ligation implement 15.

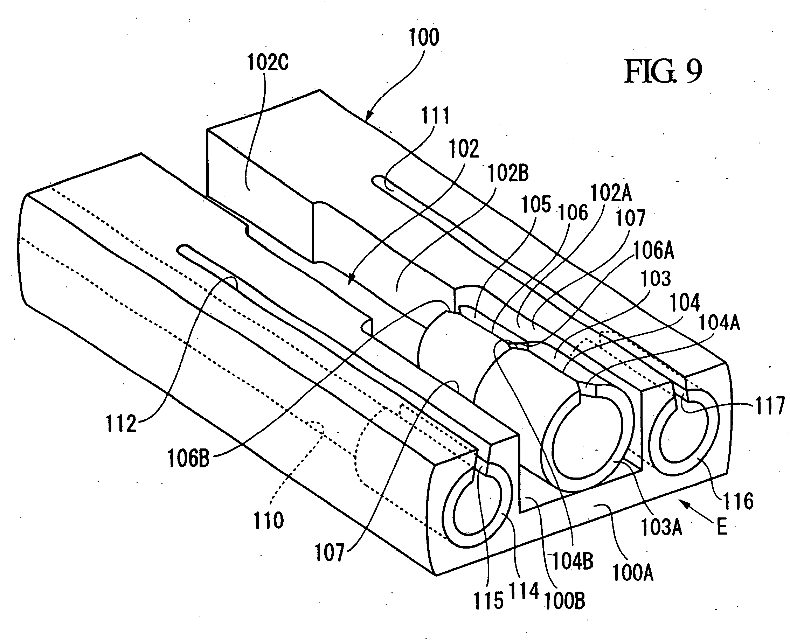

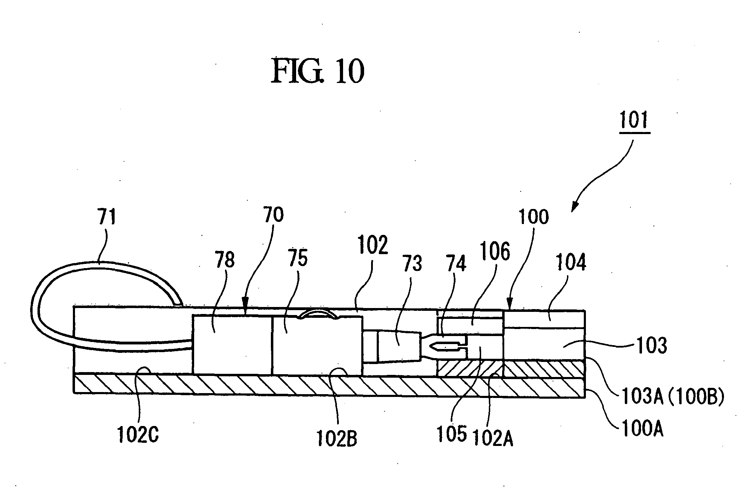

As shown in FIGS. 9 and 10, an accommodation groove 102 which is an accommodation portion in which the stopper 78 and the holding member 75 can be accommodated is formed at approximately the center portion of the accommodation container 100. The accommodation groove 102 is provided with a first groove portion 102A having a open portion 100B at one end surface 100A side of the accommodation container 100, a second groove portion 102B which reaches to the first groove portion 102A, and of which the width is smaller than the width of the first groove portion 102A, and a third groove portion 102C which reaches to the second groove portion 102B, and of which the width is smaller than the width of the second groove portion 102B. The width of the third groove portion 102C is a size at which the stopper 78 can be press-fitted into the third groove portion 102C. Moreover, an elastic member 103 for engaging is fixed in the first groove portion 102A. The elastic member 103 for engaging which is an engagement member is composed of a cylindrical member, the axis thereof is arranged so that the axis is parallel to the length of the first groove portion 102A, and the end surface 103A at one end side thereof is approximately flush with one end surface of the accommodation container 100. Furthermore, a slit 104 is formed at the upper end of the elastic member 103 for engaging along the length. One end portion 104A of the slit 104 is expanded so that the end portion 104A is expanded toward the end surface 103A of the elastic member 103 for engaging. Similarly, another end portion 104B of the slit 104 is expanded so that the end portion 104B is expanded toward the end surface of the elastic member 103 for engaging.

As shown in FIGS. 8, 10, and 11, an elastic member 105 for fixing is fixed to the inside of the first groove portion 102A so that the elastic member 105 for fixing is in contact with another end surface of the elastic member 103 for engaging. The elastic member 105 for fixing has a shape in which the diameter of the elastic member 103 for engaging is decreased. That is, a slit 106 is formed at the upper end of the cylindrical member, and one end portion 106A and another end portion 106B of the slit 106 are expanded so that the one end portion 106A and other end portion 106B are expanded toward the end surfaces of the elastic member 105 for fixing. The inner diameter of the elastic member 105 for fixing is a size at which a pair of engagement pieces 74 of the connection member 73 can be press-fitted. Therefore, the connection member 73 is fixed by the elastic member 105 for fixing, the movement thereof is regulated, and falling off of the treating implement 70 from the accommodation groove 102 is prevented. Furthermore, a clearance 107 is formed between the outer circumference of the elastic member 105 for fixing and the side wall of the first groove portion 102A, and the elastic member 105 for fixing can be elastically deformed so that the clearance of the slit 106 is expanded.

As shown in FIG. 8, a pair of engagement pieces 74 is inserted into accommodation groove 102 so that the slit 104 is arranged in the direction in which a pair of engagement pieces 74 expand. The stopper 78 is press-fitted into the third groove portion 102C so that the boundary line between the second groove portion 102B and the third groove portion 102C, and the end surface at the holding member 75 side of the stopper 78 approximately coincide with each other.

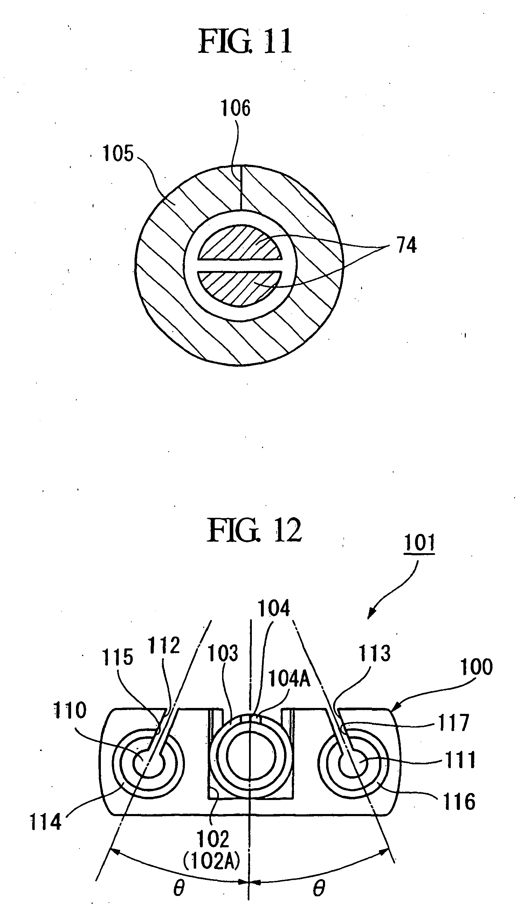

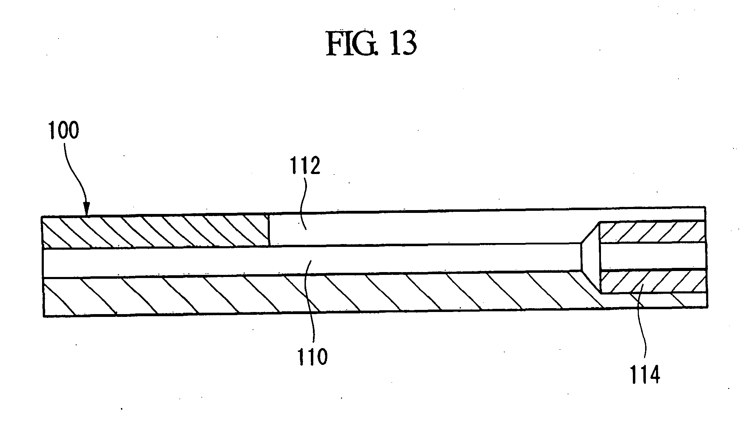

Moreover, in the accommodation container 100, two through holes 110 and 111 are formed parallel to the accommodation groove 102 so that the accommodation groove 102 is put between. The through holes 110 and 111 have the same shape, and each through hole 110 and 111 is a second engaging portion which has a diameter at which an engagement member 79 can be inserted thereinto. In the accommodation container 100, slit shaped thread seaming guides 112 and 113 are formed from the upper surface of the accommodation container 100 for each through hole 110 and 111. The seaming thread guides 112 and 113 are formed parallel to the through holes 110 and 111 from one end surface 100A of the accommodation container 100, and cease prior to the other end surface. The width of the seaming thread guides 112 and 113 is a size at which the thread 71 for seaming can be inserted. As shown in FIG. 12, in the case of being seen from the one end surface 100A side, the seaming thread guides 112 and 113 are formed so that the angle between each seaming thread guide 112 and 113 and a straight line passing through the center of the accommodation groove 102 and the slit 104 of the elastic member 103 for engaging becomes an angle θ. Furthermore, as shown in FIGS. 9 and 13, the diameter of one end portion of the through hole 110 is increased, and an elastic member 114 for engaging the needle guide is fixed to one end portion of the through hole 110. In the elastic member 114 for engaging the needle guide, a slit 115 is formed so that the slit 115 matches with the seaming thread guide 112. Similarly, an elastic member 116 for engaging a needle guide is press-fitted into one end portion of the through hole 111, and the slit 117 is formed so that a slit 117 matches with the seaming thread guide 113.

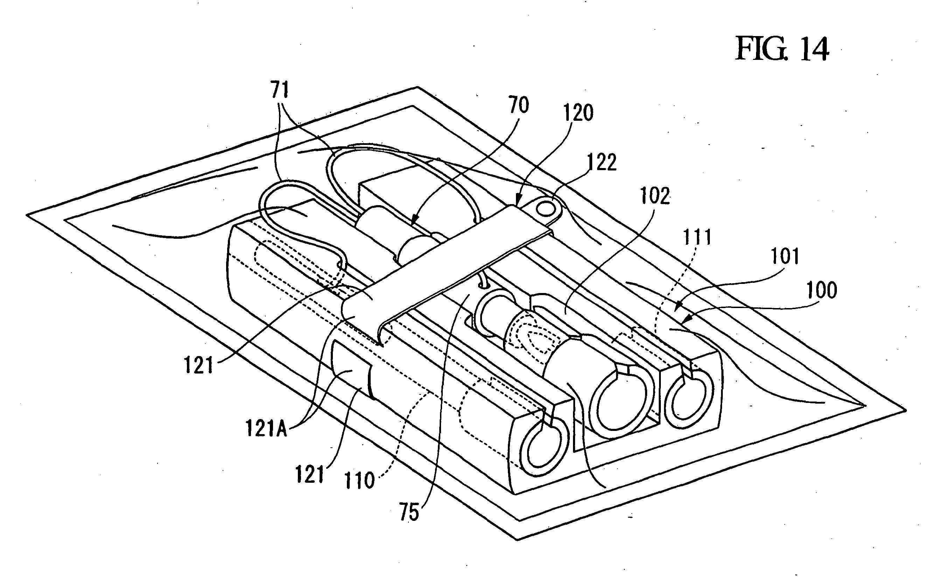

In addition, as shown in FIG. 14, in the case of preserving the treating implement cartridge 101, sterilizing packing may be performed in the state in which a fixing clip 120 is equipped. The fixing clip 120 has a pair of elastic engagement pieces 121 inserted from the side part of the accommodation container 100. The elastic engagement pieces 121 traverse the accommodation groove 102 and the through holes 110 and 111, and a folded portion 121A engaged with the side surface of the accommodation container 100 is formed at the front end thereof. The fixing clip 120 is equipped so that the elastic engagement pieces 121 push the holding member 75 from above, while the thread 71 for seaming is prevented from being pulled out from the one end surface 100A side of the accommodation container 100. A tongue portion 122 is extended from the base end portion of the elastic engagement piece 121, and by pulling the tongue portion 122, the fixing clip 120 can be easily pulled out from the accommodation container 100.

Next, the function of the present embodiment is explained.

First, the ligature and suture system for medical application 1 is mounted on the treating implement 70 in advance of operation. In the present embodiment, regarding the treating implement 70, a whole of the treating implement cartridge 101 is engaged with the ligature and suture system for medical application 1, and then, the treating implement 70 is pulled out from the accommodation container 100. In the above case, it is desirable for the fixing clip 120 to be previously removed. However, after the treating implement cartridge 101 is engaged with the ligature and suture system for medical application 1, the fixing clip 120 may be removed, and in this case, position shifting of the treating implement 70 at the time of engaging can be surely prevented.

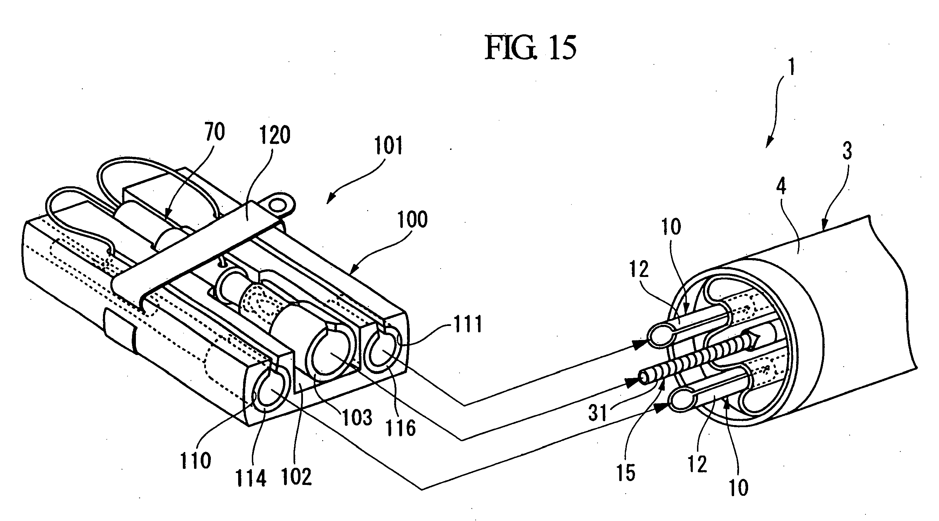

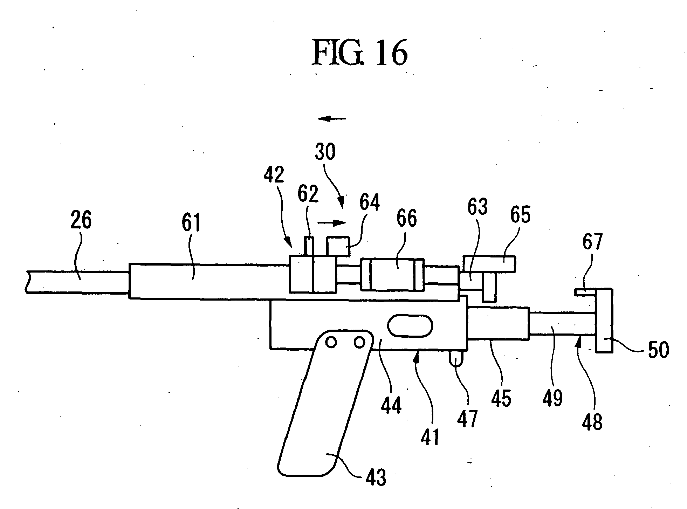

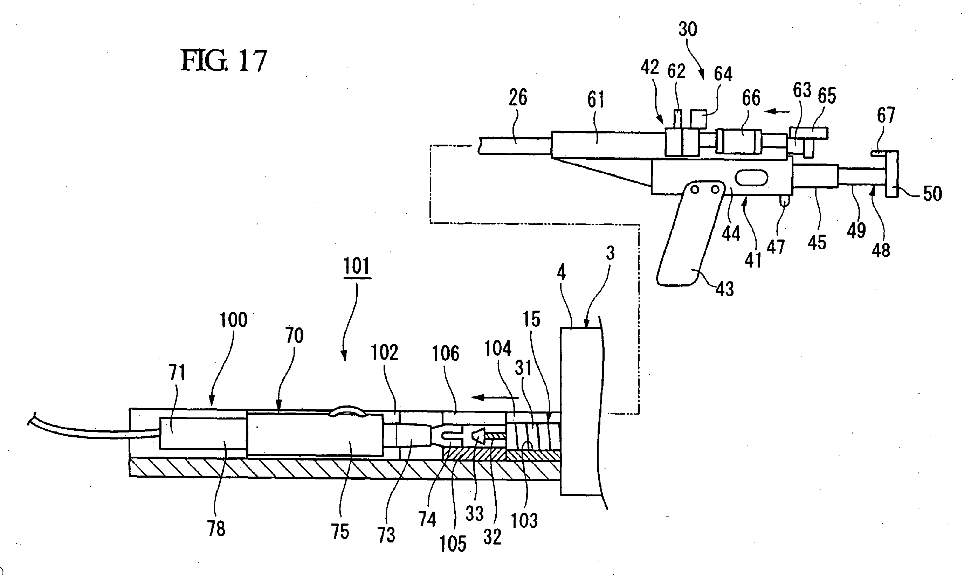

As shown in FIGS. 15 and 16, in the state in which the cutting handle 62 is put in contact with the knob portion 64, the finger control portion 65 and the ligation handle 66 are advanced along the slide guide 61, and the ligation sheath 31 is projected from the front end side of the needle guide 12. Then, the ligation handle 66 is advanced, and the arrowhead hook 33 is projected from the front end side of the ligation sheath 31. Furthermore, as shown in FIG. 17, the treating implement cartridge 101 is pushed on, and first, the front end portion of the ligation sheath 31 is press-fitted into the elastic member 103 for engaging. Thereby, if the treating implement cartridge 101 is unclasped, the treating implement cartridge 101 is not dropped out from the ligature and suture system for medical application 1.

Next, the treating implement cartridge 101 is further brought near to the ligature and suture system for medical application 1, and the front end portions of the needle guides 12 are press-fitted into the elastic members 114 and 116 for engaging the needle guides. Because the arrangement of the accommodation groove 102 of the accommodation container 100 and the penertating holes 110 and 111 coincides with the arrangement of the ligation implement 15 and the tissue through needles 10, the needle guides 12 are pulled into the insides of the through holes 110 and 111 as in this state. Here, because the elastic members 114 and 116 for engaging the needle guides are provided at the through holes 110 and 111, three portions which are two needle guides and the ligation sheath 31 are connected to the treating implement cartridge 101. In addition, the reason why the ligation sheath 31 is firstly press-fitted is that, because the ligation sheath 31 is not fixed to the over tube 3 in contrast to the needle guides 12, first, the ligation sheath 31 is fixed, then, the two needle guides 12 are inserted into the elastic members 114 and 116 for engaging the needle guides, and thereby the working becomes easy.

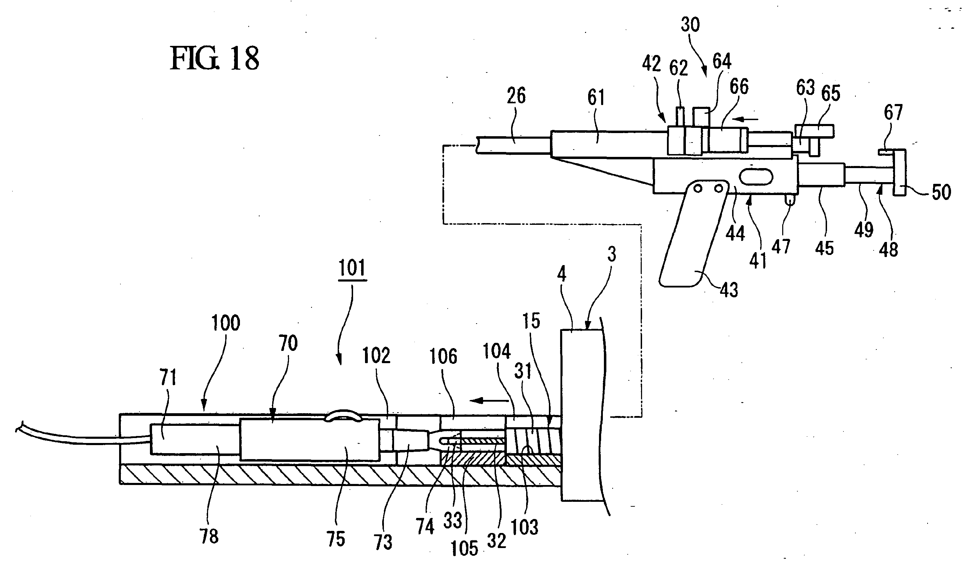

After the treating implement cartridge 101 is equipped, the connection operation with the ligation implement 15 is performed. Specifically, the ligation handle 66 of the operation portion 30 is operated, the operation wire 32 is advanced, and the arrowhead hook 33 is pulled into the inside of the elastic member 105 for fixing. At this time, elastic transformation is carried out so that the clearance 107 of the accommodation groove 102 is decreased, the elastic member 105 for fixing is pressed and expanded, the engagement piece 74 is deformed, and as shown in FIG. 18, the arrowhead hook 33 is inserted in between the pair of engagement pieces 74. Thereby, a holding force becomes weak due to elastic deformation of the elastic member 105 for fixing, on and after this, the connection member 73 can be moved along the accommodation groove 102.

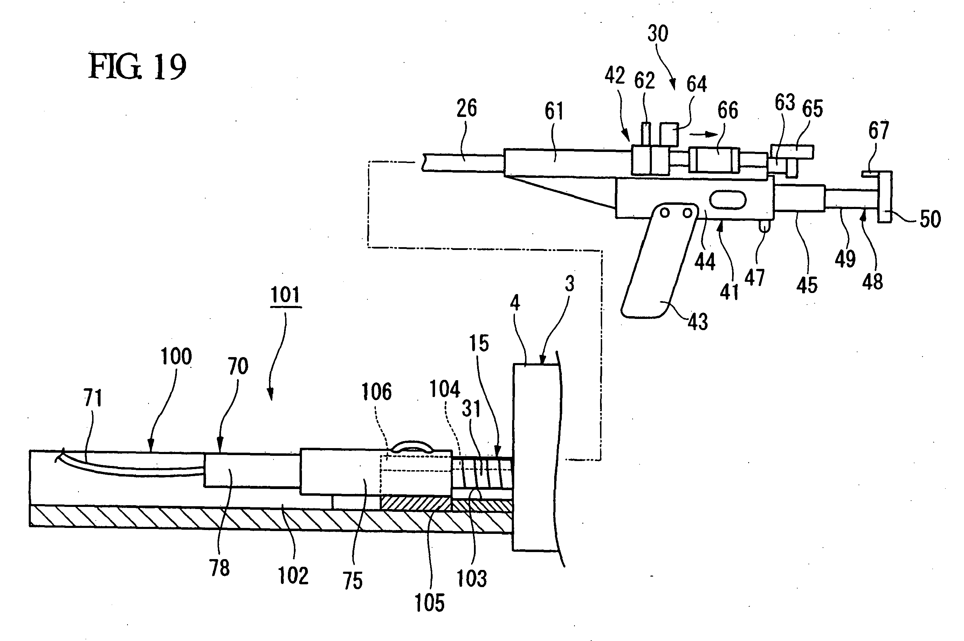

After the arrowhead hook 33 is engaged with the connection member 73, the ligation handle 66 is pulled back, and the arrowhead hook 33 is pulled into the inside of the ligation sheath 31. As shown in FIG. 19, the end surface of the holding member 75 engaged with the arrowhead hook 33 is put into contact with the front end surface of the ligation sheath 31 by operating the ligation handle 66. Thus, after the ligation implement 15 is mounted on the treating implement 70, next, the engagement member 79 is equipped.

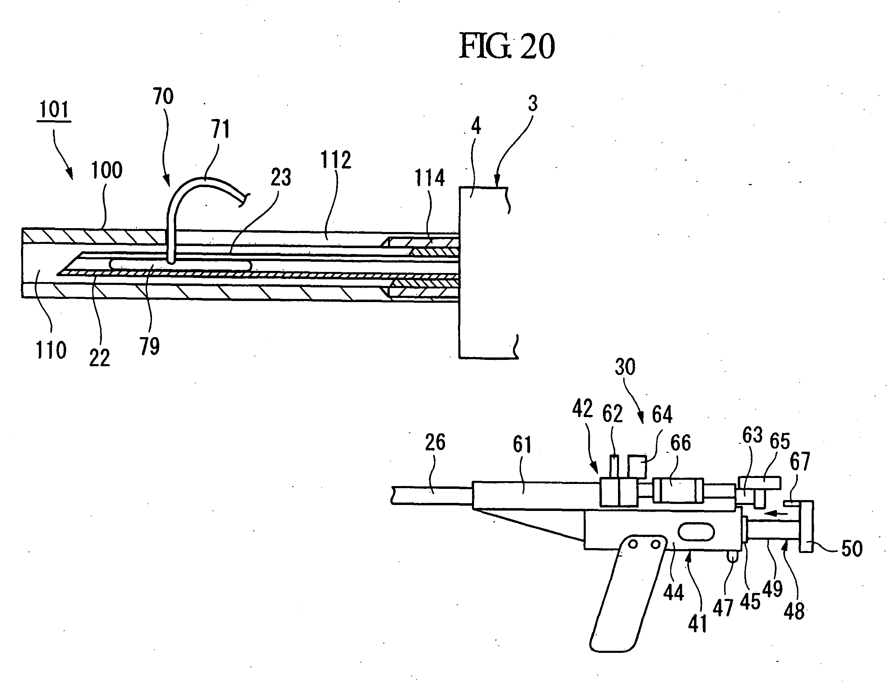

That is, as shown in FIG. 20, the tissue through needles 10 are caused to project from the needle guides 12 by operating the needle handle 45 of the operation portion 30. The hollow needles 22 at the front end of the tissue through needles 10 are inserted into the insides of the through holes 110 and 111, and the engagement members 79 accommodated in the inside of the through holes 110 and 111 are inserted into the insides of the hollow needles 22 from the front end openings of the hollow needles 22, and are accommodated. At this time, because the direction of the slits 13 of the hollow needles 22 positioned by the needle positioning pins 14 of the needle guides 12 coincide with the direction of the seaming thread guides 112 and 113 of the accommodation container 100, the thread 71 for seaming is inserted into the slits 23 of the hollow needles 22.

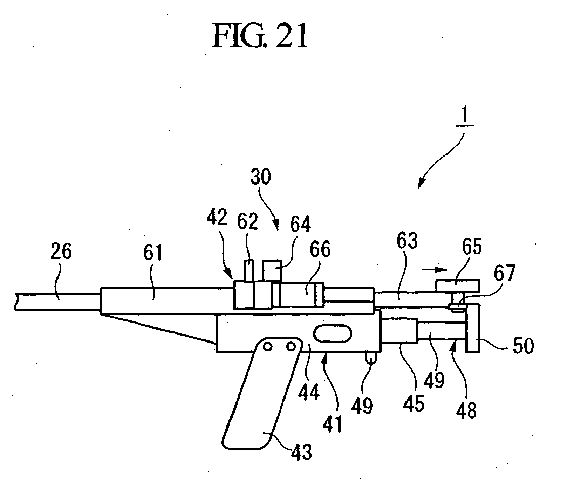

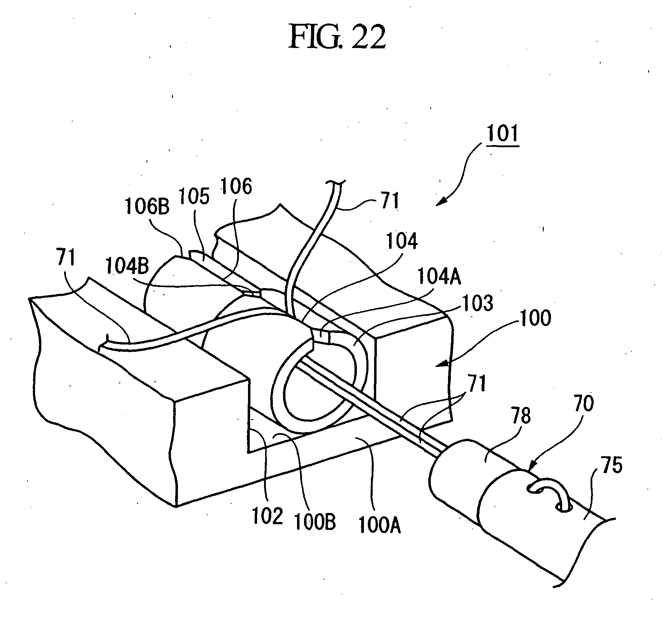

Because the treating implement 70 is mounted on the ligature and suture system for medical application 1 by the operation explained above, the treating implement 70 is drawn off from the accommodation container 100, and the treating implement 70 is accommodated on the ligature and suture system for medical application 1. First, as shown in FIG. 21, the ligation operation portion 42 is pulled back, and the ligation implement 15 is pulled back. The thread 71 for seaming engaged with the ligation implement 15 and the holding member 75 on which the thread 71 for seaming is wound are pulled. As shown in FIG. 22, the holding member 75 passes through the inside of each elastic member 103 and 105, and is pulled out from the side of the one end surface 100A of the accommodation container 100. Furthermore, the stopper 78 is pulled, and the stopper 78 is pulled out from the third groove portion 102C, and then, is pulled out from the second groove portion 102B of which the width is larger than that of the third groove portion 102C, and then is pulled out from the first groove portion 102A, in turn. The stopper 78 passes through the inside of each elastic member 103 and 105 like the holding member 75, and is pulled out from the side of the one end surface 100A of the accommodation container 100. At this time, the thread 71 for seaming pulled out from the stopper 78 is led to the slits 104 and 106 while being guided by the parts (other end portions 104B and 106B) in which the notches are formed in the slits 104 and 106 of the elastic members 103 and 105, passes through the slits 104 and 106, and is pulled out from the one end surface 100A side of the accommodation container 100.

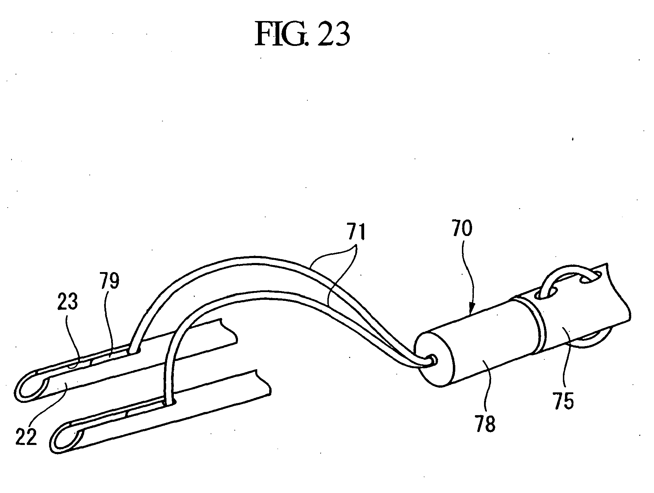

Here, because, in the operation portion 30, the finger control portion 65 of the ligation operation portion 42 and the pusher handle 48 are engaged with each other by the engagement member 67, when the ligation operation portion 42 is returned, the pusher handle 48 is pushed. Thereby, the pushers 25 are pulled back, and the spaces in which the engagement members 79 in the hollow needles 22 are accommodated are expanded to the base end side. As shown in FIG. 23, because the engagement member 79 is pulled as along with the withdrawal of the ligation implement 15, the engagement members 79 fixed to the end portions thereof are further moved to the side of the base ends of the hollow needles 22, and thereby it is difficult for the engagement members 79 to fall out from the front end openings of the hollow needles 22. When the ligation operation portion 42 is further returned, the connection pin 52 between the pusher handle 48 and the needle handle 45 is moved to the position at which both handles 45 and 48 are connected, the needle handle 45 is returned together with the pusher handle 48 and the ligation operation portion 42. Thereby, the hollow needles 22 are pulled back, and are drawn out from the side of the end surface 100A of the accommodation container 100. Thereby, the treating implement 70 is pulled out from the accommodation container 100, and is accommodated on the ligature and suture system for medical application 1. In addition, the operation portions 42 and 48 may be not connected to each other, and may be independently operated, respectively. In this case, the needle operation portion 41 is not operated, and the thread 71 for seaming pulled by the ligation implement 15 may be hooked on the rear end portions of the slits 23 of the hollow needles 22, and thereby the hollow needles 22 pulled by the thread 71 for seaming, and the hollow needles 22 pulled out from the accommodation container 100.

Thus, after the treating implement 70 is equipped, operation is started. First, the endoscope 2 is inserted into the over tube 3, and while checking the interior of the body using the image pick-up portion provided at the front end of the endoscope 2, the over tube 3 is inserted to a position at which the side hole 6 of the over tube 3 is approximately coincident with the living body tissue which is the treatment target. At this position, using the gripping forceps inserted into the endoscope 2, or the suction unit, the living body tissue is pulled into the inside of the chamber 5 from the side hole 6.

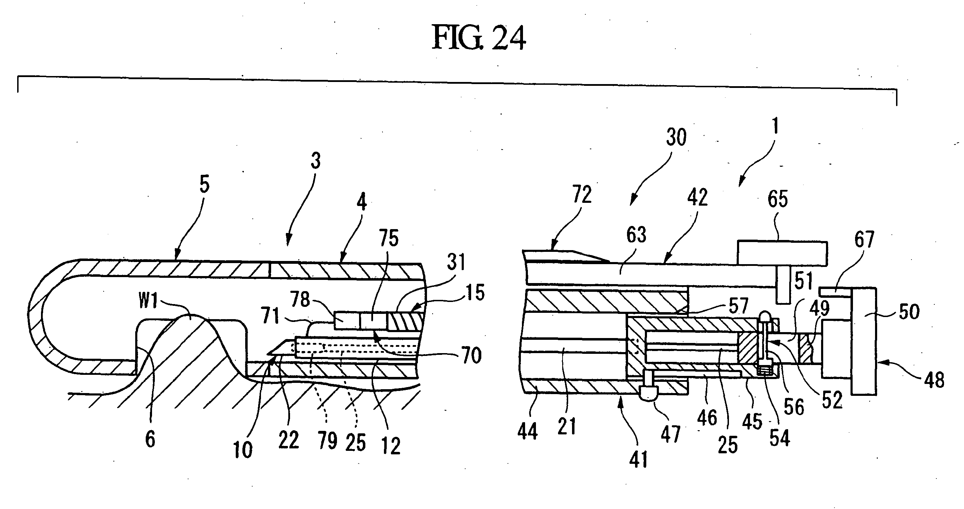

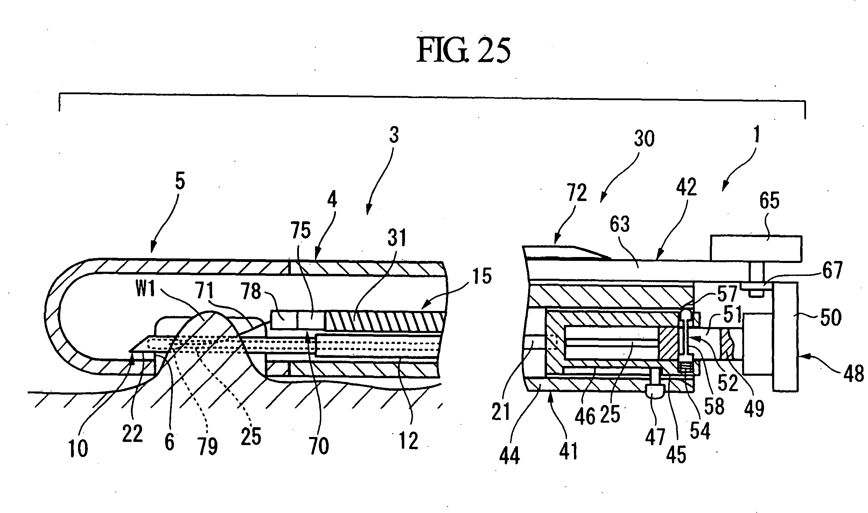

Furthermore, as shown in FIG. 24, the living body tissue W1 is penetrated by the tissue through needles 10. Specifically, the pusher handle 48 is pressed to the accommodation portion 44 side. Because, in the initial state, the needle handle 45 is connected to the pusher handle 48 by the connection pin 52, the entire tissue through needles 10 are protruded. As shown in FIG. 25, the hollow needles 22 are projected from the needle guides 12, and penetrate from the base end side to the front end side of the living body tissue W1, and along with this, the thread 71 for seaming also penetrates into the living body tissue W1. In this case, because the ligation operation portion 42 engaged with the handle body 50 of the pusher handle 48 is also pressed, the whole of the ligation implement 15 is protruded, and the distance between the engagement member 79 and the stopper 78 does not change. Therefore, too much tension is not created on the thread 71 for seaming.

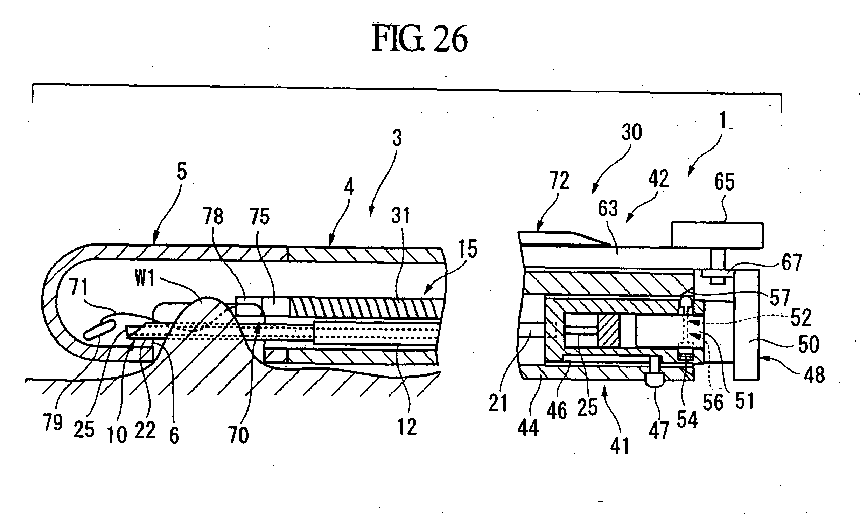

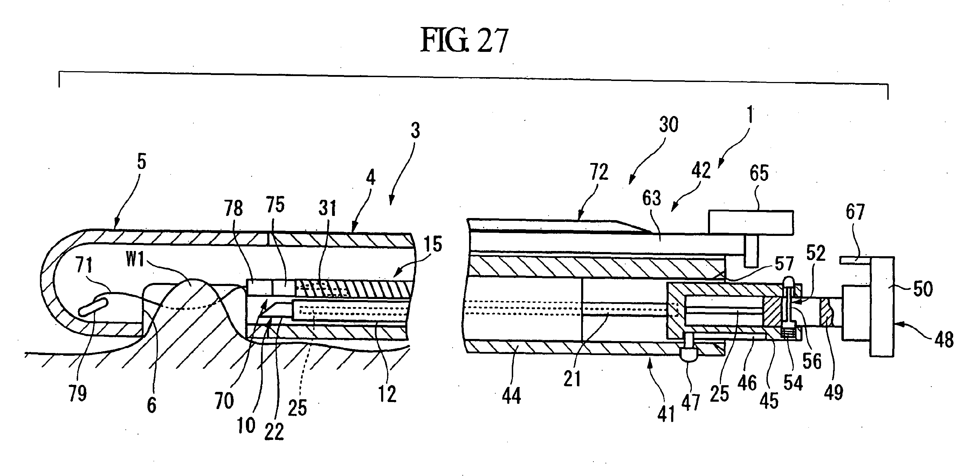

In the process in which the pusher handle 48 is pressed, when the connection pin 52 is accommodated in the inside of the accommodation portion 44, the connection pin 52 is moved, and the connection with the needle handle 45 is released. On and after this, in the state in which the needle handle 45 is stopped, that is, in the state in which the hollow needles 22 are fixed, only the pusher handle 48 is pressed, and only the pusher 25 is moved. As a result, as shown in FIG. 26, the engagement members 79 are pushed out from the front end openings of the hollow needles 22 by the pushers 25. After the engagement members 79 are released, the pusher handle 48 is returned, and the pushers 25 are returned to the inside of the hollow needles 22. As shown in FIG. 27, in the process in which the pusher handle 48 is pulled back, the connection pin 52 moves again and returns to the connection position. On and after this, the pusher handle 48 and the needle handle 45 are connected to each other, the entire tissue through needles 10 are pulled back, and the hollow needles 22 are pulled out from the living body tissue W1. In addition, the engagement members 79 are held at the front end side of the living body tissue W1, and the thread 71 for seaming is maintained in the state in which the living body tissue W1 is penetrated. Furthermore, because the engagement between the pusher handle 48 and the ligation operation portion 42 is released in the direction in which the pusher handle 48 is returned, the ligation implement 15 does not move.

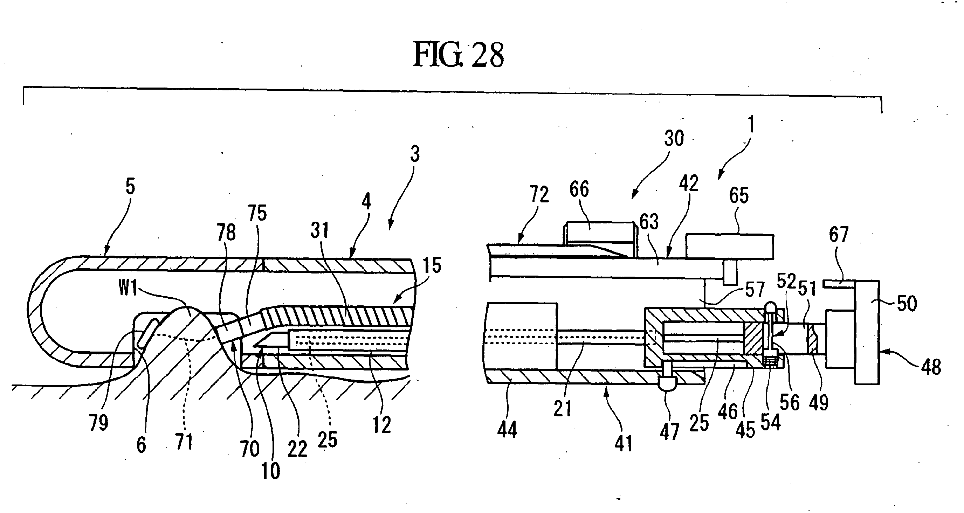

Next, in the state in which the finger is put on the finger control portion 65 of the ligation operation portion 42, and the operation portion body 63 is fixed, the ligation handle 66 is pulled back, and the thread 71 for seaming is pulled toward the operator by the arrowhead hook 33 attached to the operation wire 32. As shown in FIG. 28, because the holding member 75 is in contact with the front end surface of the ligation sheath 31, and does not move, the distance between the stopper 78 and the engagement members 79 is reduced, and ligation is carried out while the living body tissue W1 is put between the engagement members 79 and the stopper 78. Furthermore, the cutting handle 62 is pressed, and the sheath 34 for cutting is protruded along the ligation sheath 31. The cutting member 35 provided at the front end of the sheath 34 for cutting is protruded while covering the outer circumference of the holding member 75, and cuts the thread 71 for seaming pulled out to the outer circumference of the holding member 75 by the cutting portion 36 provided at the front end thereof. When the cutting handle 62 is returned, and then a whole of the ligation operation portion 42 is returned, the part from the cut end portion of the thread 71 for seaming to the holding member 75 and the engagement member 79 are separated, and held at the side of the living body tissue W1. The holding member 75 falls out from the thread 71 for seaming and the ligation implement 15, and is discharged to the outside of the body.

According to the present embodiment, because the accommodation groove 102 and the through holes 110 and 111 which separately accommodate the connection member 73 and the engagement members 79 of the treating implement 70 are provided, by inserting the ligation implement 15 and the hollow needles 22 into them, respectively, the treating implement 70 can be mounted on the ligature and suture system for medical application 1. Therefore, the equipping operation of the treating implement 70 becomes easy, and the time of performing the operation can be shortened.

Because the elastic member 103 for engaging and the elastic members 114 and 116 for engaging the needle guides are provided in the accommodation groove 102 and the through holes 110 and 111, respectively, the ligation implement 15 and the hollow needles 22 can be engaged with the treating implement cartridge 101, and thereby position shifting during the equipping operation can be prevented. Moreover, the treating implement 70 can be equipped alone.

Because, in the treating implement cartridge 101, the stopper 78 is press-fitted into the third groove portion 102C, the engagement piece 74 of the connection member 73 is accommodated in the inside of the elastic member 105 for fixing, and the engagement members 79 are inserted into the inside of the through holes 110 and 111, and thereby the movement of each part of the treating implement 70 is regulated, preservation of the treating implement 70 becomes easy, and the equipment for the ligature and suture system for medical application becomes easy. Furthermore, sterilizing becomes easy. Here, the fixing clip 120 is used, and thereby the preservation and the equipment of the treating implement 70 become easier and simpler.

Because the elastic member 105 for fixing is provided separated from the wall surface with the second groove portion 102B, and the elastic member 105 for fixing is easy to deform so that the slit 106 is opened, the arrowhead hook 33 is easy to engage with the engagement piece 74 of the connection member 73. Here, if the connection member 73 is held so that the slit 106 and a pair of engagement pieces 74 are stood in line on one straight line, the engagement piece 74 is easy to deform in the case in which the arrowhead hook 33 is inserted.

Because the slits 104 and 106 through which the thread 71 for seaming passes are provided at each elastic member 103 and 105, when the treating implement 70 is pulled away from the accommodation container 100, the thread 71 for seaming can be simply pulled out.

Because the seaming thread guides 112 and 113 and the slits 23 of the hollow needles 22 are coincident, the hollow needles 22 are alone protruded, while accommodating the engagement member 79, and the thread 71 for seaming can be pulled out from the hollow needles 22.

In addition, the present invention can be applied widely without being limited to the above-mentioned embodiment.

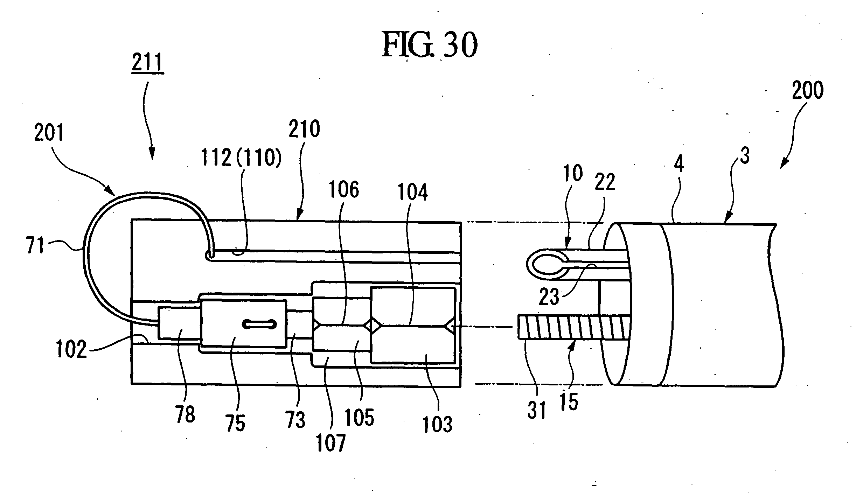

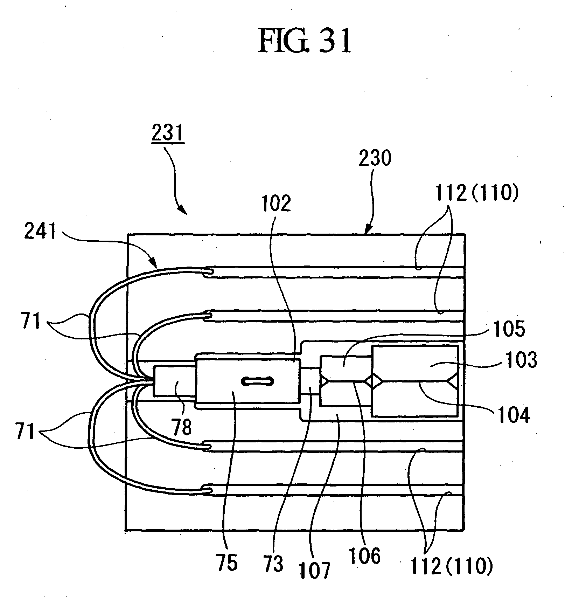

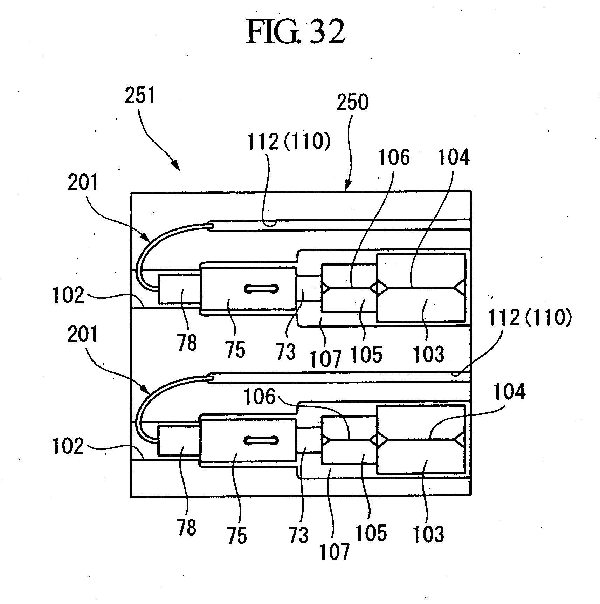

For example, as shown in FIG. 30, in the case of a ligature and suture system 200 for medical application provided with one tissue through needle 10 and one ligation implement 15, the constitution of the treating implement 201 is that the thread 71 for seaming fixed to one engagement member 79 is passed through the stopper 78, and is held by the holding member 75. According to this, the treating implement cartridge 211 becomes a accommodation container 210 provided with one accommodation groove 102, and one through hole 110. Moreover, as in the treating implement cartridge 231 shown in FIG. 31, an accommodation container 230 may be provided with two through holes at each of both sides of one accommodation groove 102, that is, with a total of four through holes. The treating implement 241 in such a case is provided with four engagement members 79, one stopper 78, a connection member 73, and a holding member 75. Such a treating implement cartridge 231 is applied to a ligature and suture system for medical application provided with four tissue through needles 10 for one ligation implement 15. Furthermore, as in the treating implement cartridge 251 shown in FIG. 32, an accommodation container 250 may be provided with two accommodation grooves 102 and two through holes 110. The accommodation grooves 102 and the through holes 110 are arranged by turns, and one treating implement 210 is accommodated for one accommodation groove 102 and one through hole 110. Such a treating implement cartridge 251 is applied to a ligature and suture system for medical application provided with two sets of ligation implements 15 and two sets of tissue through needles 10. The function and effects of the treating implement cartridges 211, 231, and 251, the treating implements 201 and 241, and the ligature and suture systems 200 for medical application are the same as above.

In the treating implement cartridge for living body tissue according to the present invention, the engagement portion of the treating implement is accommodated in the accommodation container, the engaged portion at the side of the treating apparatus is inserted into the accommodation container from the opening portion of the accommodation container, and the treating apparatus is operated, and thereby, in the state in which the treating implement is accommodated in the accommodation container, the engaging portion and the engaged portion are engaged with each other, and the treating implement is mounted on the treating apparatus. Because at least one engaging portion of the treating implement is fixed by the fixing portion, the treating implement is prevented from slipping out from the accommodation container, and the corresponding engaged portion is easily inserted.

In the treating implement cartridge for living body tissue according to the present invention, a plurality of engagement portions of the treating implement are connected via the flexible member, and each engagement portion is accommodated in the accommodation container. When being mounted on the treating apparatus, an engaged portion corresponding to each engaging portion is engaged. In this case, when the first engaging portion is pulled, the second engaging portion connected to the flexible member also is moved accompanying this, and the moving direction thereof is the direction in which the engagement state with the second engaged portion is maintained.

In the present invention, the fixing portion can fix the first engaging portion in a direction in which the first engaged portion is pressed, and is engaged, and in an inverse direction thereof, and the accommodation container can fix the second engaging portion in a direction in which the second engaged portion is pressed, and is engaged.

In the present treating implement cartridge for living body tissue, the second engaging portion is fixed in the direction in which the second engaged portion is inserted and is engaged, and thereby the engagement operation is assisted. Moreover, the fixing portion holds the first engaging portion so that the first engaging portion is not moved until the first engaging portion is engaged with the first engaged portion, and after the first engaging portion is engaged, the fixing portion is pulled out from the first engaging portion by the movement of the first engaged portion, and thereby the engagement operation is assisted, and after the treating implement is mounted on the treating apparatus, the treating implement can be removed from the accommodation container.

In the present invention, the flexible member is a thread for seaming, the second engaged portions are hollow needles by which the living body tissue is penetrated, and the second engaging portions are engagement members arranged in the hollow needles, and, after the hollow needles penetrate into the living body tissue, these are released from the inside of the hollow needles, and make the flexible member engage with the living body tissue.

In the present treating implement cartridge of the living body tissue, the second engaging portions of the treating implement are the engagement members, and the engagement members thereof are engaged with the treating apparatus in the case of being accommodated in the hollow needles. In the case of using the treating implement, after the hollow needles penetrate into the living body tissue, the engagement members are pulled out from the hollow needles, and the engagement is released.

In the present invention, an engagement portion which is engaged with the treating apparatus inserted into the accommodation portion is provided at the opening portion of the accommodation container.

In the treating implement cartridge of the present invention, when the engaging portion is inserted into the accommodation portion, and is engaged with the engaged portion of the treating apparatus, the treating apparatus can be engaged with the engaging portion. For this reason, when the treating implement is mounted on the treating apparatus, the operation can be performed in the state in which the treating apparatus is engaged with the accommodation container.

According to the present invention, in the state in which a plurality of engaging portions provided to the treating implement are held by one accommodation container, a plurality of engaged portions at the side of the treating apparatus are received, the corresponding engaging portions and the engaged portions can be engaged with each other, and thereby equipment operation can be surely and easily performed. Especially, because the position of each engaging portion can be previously set at a specific position by the accommodation container, the positioning operation becomes easy. Moreover, because, when one engaging portion is engaged, and then, other engaging portions are engaged, the engagement state of the engaging portion which has been already engaged can be maintained, the equipment operation can be performed alone, and the operation also becomes easy in the case performance of equipment operation by a plurality of men.

## Claims

1. A ligating implement cartridge (101) for use in ligating living body tissue, comprising:
a ligating implement (70) which has a plurality of engaging portions (74, 79) adapted to engage with a plurality of engaged portions (33, 22) provided at a front end portion of a treatment apparatus (1) to be inserted into an interior of a body to perform a ligation treatment of the living body tissue according to operation of the treatment apparatus (1) by an operator;
an accommodation container (100) which accommodates the ligating implement (70) prior to its engagement with the front end portion of the treatment apparatus (1), and from which the ligating implement (70) is drawn off after the ligating implement (70) is engaged with the front end portion of the treatment apparatus (1), the accommodation container (100) having a plurality of accommodation portions (102A, 102B, 102C) which accommodate the engaging portions (74, 79) of the ligating implement (70), and which have opening portions (100B) into which the engaged portions (33, 22) are to be inserted; and
a fixing portion (105) which is attached to at least one of the accommodation portions (102), and holds one of the engaging portions (74, 79).

2. A ligating implement cartridge (101) according to claim 1, wherein the ligating implement (70) comprises:
a first engaging portion (74) from among the plurality of engaging portions adapted to engage with a first engaged portion (33) from among the plurality of engaged portions;
a second engaging portion (79) from among the plurality of engaging portions adapted to engage with a second engaged portion (22) from among the plurality of engaged portions; and
a flexible member (71) which connects the first engaging portion (74) and the second engaging portion (79),
wherein the second engaging portion (79) is adapted to be pulled via the flexible member (71) when the first engaged portion (33) which is attached to the first engaging portion (74) is relatively moved with respect to the second engaged portion (22), and an engagement state between the second engaging portion (79) and the second engaged portion (22) is maintained.

3. A ligating implement cartridge (101) according to claim 2,
wherein the fixing portion (105) fixes the first engaging portion (74) in a direction in which the first engaged portion (33) is adapted to be pushed and engaged, and an inverse direction thereof, and the accommodation container (100) fixes the second engaging portion (79) in a direction in which the second engaged portion (22) is adapted to be pushed and engaged.

4. A ligating implement cartridge (101) according to claim 3,
wherein the flexible member (71) is a thread for seaming, the second engaged portion (22) is a hollow needle with which the living body tissue can be penetrated, and the second engaging portion (79) is an engagement member which is arranged in an inside of the hollow needle (22), wherein, after the hollow needle (22) penetrates into the living body tissue, the second engaging portion (79) is adapted to be released from the inside of the hollow needle (22), and to make the flexible member (71) engage with the living body tissue.

5. A ligating implement cartridge (101) according to claim 1,
wherein an engagement portion (103) which is engaged with the treatment apparatus (1) inserted into the accommodation portion (102) is provided at an opening portion (100B) of the accommodation container (100).

## Patentansprüche

1. Patrone (101) eines Ligaturgeräts zur Verwendung bei der Ligatur von Gewebe eines lebenden Körpers, aufweisend:
ein Ligaturgerät (70), das eine Mehrzahl aufnehmende Eingriffsabschnitte (74, 79) hat, die für den Eingriff mit einer Mehrzahl aufgenommener Eingriffsabschnitt (33, 22) am vorderen Endabschnitt einer Behandlungsvorrichtung (1) ausgeführt sind, die in das Innere eines Körpers einzuführen ist, um eine Ligaturbehandlung des Gewebes des lebenden Körpers entsprechend der Betätigung der Behandlungsvorrichtung (1) durch einen Operateur auszuführen;
einen Aufnahmebehälter (100), der das Ligaturgerät (70) vor dem Eingriff mit dem vorderen Endabschnitt der Behandlungsvorrichtung (1) aufnimmt, und aus dem das Ligaturgerät (70) herausgezogen wird, nachdem das Ligaturgerät (70) mit dem vorderen Endabschnitt der Behandlungsvorrichtung (1) in Eingriff gebracht worden ist, wobei der Aufnahmebehälter (100) eine Mehrzahl Aufnahmeabschnitte (102A, 102B, 102C), die die aufnehmenden Eingriffsabschnitte (74, 79) des Ligaturgeräts (70) aufnehmen, und Öffnungsabschnitte (100B) hat, in die die aufgenommenen Eingriffsabschnitte (33, 22) einzuführen sind; und
einen Befestigungsabschnitt (105), der an mindestens einem der Aufnahmeabschnitte (102) angebracht ist, und einen der aufnehmenden Eingriffsabschnitte (74, 79) hält.

2. Patrone (101) eines Ligaturgeräts nach Anspruch 1, bei der das Ligaturgerät (70) aufweist:
einen ersten aufnehmenden Eingriffsabschnitt (74) der Mehrzahl aufnehmender Eingriffsabschnitte, der zum Eingriff mit einem ersten aufgenommenen Eingriffsabschnitt (33) der Mehrzahl aufgenommener Eingriffsabschnitte eingerichtet ist;
einen zweiten aufnehmenden Eingriffsabschnitt (79) der Mehrzahl aufnehmender Eingriffsabschnitte, der zum Eingriff mit einem zweiten aufgenommenen Eingriffsabschnitt (22) der Mehrzahl aufgenommener Eingriffsabschnitte eingerichtet ist; und
ein flexibles Element (71), das den ersten aufnehmenden Eingriffsabschnitt (74) und den zweiten aufnehmenden Eingriffsabschnitt (79) verbindet,
wobei der zweite aufnehmende Eingriffsabschnitt (79) so ausgeführt ist, dass er über das flexible Element (71) gezogen wird, wenn der erste aufgenommene Eingriffsabschnitt (33), der am ersten aufnehmenden Eingriffsabschnitt (74) angebracht ist, bezüglich des zweiten aufgenommenen Eingriffsabschnitts (22) relativ bewegt wird, und ein Eingriffszustand zwischen dem zweiten aufnehmenden Eingriffsabschnitt (79) und dem zweiten aufgenommenen Eingriffsabschnitt (22) aufrechterhalten wird.

3. Patrone (101) eines Ligaturgeräts nach Anspruch 2,
bei der der Befestigungsabschnitt (105) den ersten aufnehmenden Eingriffsabschnitt (74) in der Richtung fixiert, in der der erste aufgenommene Eingriffsabschnitt (33) geschoben und in Eingriff gebracht werden kann sowie in der umgekehrten Richtung, und der Aufnahmebehälter (100) den zweiten aufnehmenden Eingriffsabschnitt (79) in der Richtung fixiert, in der der zweite aufgenommene Eingriffsabschnitt (22) geschoben und in Eingriff gebracht werden kann.

4. Patrone (101) eines Ligaturgeräts nach Anspruch 3,
bei der das flexible Element (71) ein Faden zum Nähen, der zweite aufgenommene Eingriffsabschnitt (22) eine Hohlnadel, mit der das Gewebe des lebenden Körpers durchdrungen werden kann, und der zweite aufnehmende Eingriffsabschnitt (79) ein Eingriffselement ist, das im Innern der Hohlnadel (22) angeordnet ist, wobei der zweite aufnehmende Eingriffsabschnitt (79) so ausgeführt ist, dass er aus dem Innern der Hohlnadel (22) freigegeben werden kann und das flexible Element (71) mit dem Gewebe des lebenden Körpers in Eingriff bringt.

5. Patrone (101) eines Ligaturgeräts nach Anspruch 1,
bei der ein Eingriffsabschnitt (103), der mit der in den Aufnahmeabschnitt (102) eingeführten Behandlungsvorrichtung (1) in Eingriff steht, am Öffnungsabschnitt (100B) des Aufnahmebehälters (100) vorgesehen ist.

## Revendications

1. Cartouche (101) pour un instrument de ligature destinée à être utilisée pour ligaturer un tissu d'un corps vivant, comprenant :
un instrument de ligature (70) qui comporte une pluralité de parties d'engagement (74, 79) adaptées pour s'engager avec une pluralité de parties engagées (33, 22) prévues au niveau d'une partie d'extrémité avant d'un appareil de traitement (1) destiné à être inséré dans un intérieur d'un corps pour effectuer un traitement de ligature du tissu du corps vivant selon l'utilisation de l'appareil de traitement (1) par un opérateur ;
un récipient de réception (100) qui reçoit l'instrument de ligature (70) avant son engagement avec la partie d'extrémité avant de l'appareil de traitement (1), et duquel l'instrument de ligature (70) est sorti après que l'instrument de ligature (70) a été engagé avec la partie d'extrémité avant de l'appareil de traitement (1), le récipient de réception (100) comportant une pluralité de parties de réception (102A, 102B, 102C) qui reçoivent les parties d'engagement (74, 79) de l'instrument de ligature (70), et qui présentent des parties d'ouverture (100B) dans lesquelles les parties engagées (33, 22) sont destinées à être insérées ; et
une partie de fixation (105) qui est attachée à au moins une des parties de réception (102) et maintient une des parties d'engagement (74, 79).

2. Cartouche (101) pour un instrument de ligature selon la revendication 1, dans laquelle l'instrument de ligature (70) comprend :
une première partie d'engagement (74) parmi la pluralité de parties d'engagement adaptée pour s'engager avec une première partie engagée (33) parmi la pluralité de parties engagées ;
une deuxième partie d'engagement (79) parmi la pluralité de parties d'engagement adaptée pour s'engager avec une deuxième partie engagée (22) parmi la pluralité de parties engagées ; et
un élément flexible (71) qui connecte la première partie d'engagement (74) et la deuxième partie d'engagement (79),
dans laquelle la deuxième partie d'engagement (79) est adaptée pour être tirée par l'intermédiaire de l'élément flexible (71) lorsque la première partie engagée (33) qui est attachée à la première partie d'engagement (74) est déplacée relativement par rapport à la deuxième partie engagée (22), et un état d'engagement entre la deuxième partie d'engagement (79) et la deuxième partie engagée (22) est maintenu.

3. Cartouche (101) pour un instrument de ligature selon la revendication 2,
dans laquelle la partie de fixation (105) fixe la première partie d'engagement (74) dans une direction dans laquelle la première partie engagée (33) est adaptée à être poussée et engagée, et une direction inverse à celle-ci, et le récipient de réception (100) fixe la deuxième partie d'engagement (79) dans une direction dans laquelle la deuxième partie engagée (22) est adaptée à être poussée et engagée.

4. Cartouche (101) pour un instrument de ligature selon la revendication 3,
dans laquelle l'élément flexible (71) est un fil de couture, la deuxième partie engagée (22) est une aiguille creuse avec laquelle le tissu du corps vivant peut être pénétré, et la deuxième partie d'engagement (79) est un élément d'engagement qui est agencé dans un intérieur de l'aiguille creuse (22), dans laquelle, après que l'aiguille creuse (22) a pénétré dans le tissu du corps vivant, la deuxième partie d'engagement (79) est adaptée pour être libérée de l'intérieur de l'aiguille creuse (22), et pour faire en sorte que l'élément flexible (71) soit engagé avec le tissu du corps vivant.

5. Cartouche (101) pour un instrument de ligature selon la revendication 1,
dans laquelle une partie d'engagement (103) qui est engagée avec l'appareil de traitement (1) inséré dans la partie de réception (102) est prévue au niveau d'une partie d'ouverture (100B) du récipient de réception (100).
